# EUROPEAN PATENT APPLICATION

(11) **EP 3 388 000 A1**
(43) Date of publication of application: **17.10.2018**
(21) Application number: 18174858.3
(22) Date of filing: 13.03.2012
(51) Int. Cl.: A61B 17/072

(54) **SURGICAL STAPLE CARTRIDGES AND END EFFECTORS WITH VESSEL MEASUREMENT ARRANGEMENTS**

(30) Priority: 15.03.2011 US 201113048590
(62) Divisional of application: 12713442.7
(71) Applicant: Ethicon Endo-Surgery, Inc., Cincinnati, OH 45242 (US)
(72) Inventor: O'CONNOR, Megan A., Cincinnati, Ohio 45227 (US); KRUTH, Robert P., Plainsboro, New Jersey 08536 (US); SWAYZE, Jeffrey S., Hamilton, Ohio 45011 (US)
(74) Representative: Tunstall, Christopher Stephen

(57) **Abstract**

Surgical staple cartridges and end effectors for assessing a size of tissue stapled by the staple cartridge or otherwise manipulated by the end effector. In various forms, the staple cartridge has at least one series of measurement indicia on the deck base that may be viewed from above the desk surface when the anvil is clamping the tissue onto the staple cartridge. Other embodiments include structure for forming a mark on the cartridge deck that corresponds to a size of the tissue supported thereon. Methods for severing and stapling tissue are also disclosed.

## Description

### BACKGROUND

### Technical Field

The present invention relates to surgical instruments and, in various embodiments, to surgical end effectors used to manipulate and/or treat tissue as well as surgical stapling instruments, end effectors and staple cartridges therefor that are designed to cut and staple tissue.

### Background

Surgical cutting and stapling instruments have been used to simultaneously make a longitudinal incision in tissue and apply lines of staples on opposing sides of the incision. Such instruments commonly include a pair of cooperating jaw members that, if the instrument is intended for endoscopic or laparoscopic applications, are capable of passing through a cannula passageway. One of the jaw members commonly supports a staple cartridge that operably supports at least two laterally spaced rows of unformed staples therein. The other jaw member defines an anvil that has staple-forming pockets that, when the anvil is closed, are aligned with rows of unformed staples supported in the cartridge.

In use, a clinician is able to close the jaw members of the cutting and stapling instrument upon tissue to position the tissue prior to actuating the instrument. Once the clinician has determined that the jaw members are properly gripping the target tissue, the clinician can then actuate or "fire" the instrument, thereby severing and stapling the tissue. The simultaneous severing and stapling avoids complications that may arise when performing such actions sequentially with different surgical tools that respectively only sever or staple.

A variety of surgical cutting and stapling instruments are known that may be employed laparoscopically and/or in connection with various "open" surgical procedures. Some surgical cutting and stapling instruments are configured to actuate replaceable cartridges that support the unformed staples therein. Such devices commonly employ a retractable cutting member that remains with the stapling instrument and may be reused with several cartridges. After the staples are fired in one cartridge, the cutting member is retracted and the spent cartridge is removed to enable a new cartridge to be installed if desired. As the cutting member is driven distally through the cartridge, the unformed staples are fired out of their respective pockets in the cartridge into forming contact with the underside of the anvil. Examples of such devices are disclosed in U.S. Patent No. 7,000,818, entitled "Surgical Stapling Instrument Having Separate Distinct Closing and Firing Systems", issued February 21, 2006, the disclosure of which is herein incorporated by reference in its entirety.

Other surgical cutting and stapling instruments employ what is commonly referred to as a "disposable loading unit" or "DLU". Such devices support a staple cartridge and a fresh knife in the form of a "unit" that is configured to be operably attached to the cutting and stapling instrument. The units are designed to be discarded after the staples have been fired. Examples of such instruments are disclosed in U.S. Patent No. 5,865,361 entitled "Surgical Stapling Apparatus", issued February 2, 1999, the entire disclosure of which is herein incorporated by reference.

In many surgical procedures and, in particular, in many vascular-related surgical procedures, it may be advantageous for the surgeon to know the size (i.e., diameter) of the vessel being transected and stapled for hemostatic purposes. Some of the existing surgical cutting and stapling devices provide measurement lines on the lateral sides of a channel that supports the surgical staple cartridge. However, such measurement lines are typically only visible from the sides of the end effector and thereby may be obscured by the vessel being cut and stapled. If the surgeon views the end effector from above, such measurement lines are not viewable and may be of little use in determining the size of the vessel being stapled. Accordingly, there is a need for surgical staple cartridge and end effector arrangements that provide feedback concerning the size of tissue being transected and stapled.

A variety of other end effector arrangements are also used by surgeons to manipulate tissue. Such devices, for example surgical grasping devices, include movable jaws for grasping tissue therebetween. It would also be advantageous for such devices and others used to treat/manipulate vascular tissue and the like to provide feedback to the user concerning the size of the tissue being treated/manipulated.

The foregoing discussion is intended only to illustrate some of the shortcomings present in the field of the invention at the time, and should not be taken as a disavowal of claim scope.

### SUMMARY

In accordance with general aspects of at least one form, there is provided a surgical staple cartridge that includes a cartridge body that operably supports a plurality of surgical staples therein. The cartridge body has a proximal end and a distal end and a deck that extends therebetween. Tissue measurement means are provided on the deck for determining a size of tissue placed thereon.

In accordance with other general aspects of at least one form, there is provided a surgical end effector for use with a surgical instrument. In various forms, the end effector includes an elongated channel that is operably couplable to the surgical instrument. A staple cartridge that has a cartridge body is operably supported in the elongated channel. The cartridge body has a deck surface that is substantially split into a first deck portion and a second deck portion by a longitudinal slot that extends therebetween. The cartridge body operably supports a first plurality of unformed staples therein that correspond to the first deck portion. The cartridge body further operably supports a second plurality of unformed staples that correspond to the second deck portion. A tissue cutting member is operably supported in the cartridge body for axial advancement in the longitudinal slot upon application of a cutting actuation motion thereto by the surgical instrument. An anvil is supported for movable travel toward and away from the deck surface in response to opening and closing motions applied thereto by the surgical instrument. Measurement means is provided on at least one of the deck surface and the anvil for determining a size of tissue placed thereon.

In accordance with still other general aspects of at least one form, there is provided a method for severing and stapling tissue. In various forms, the method comprises clamping a piece of the tissue between a surgical staple cartridge and an anvil and forming a mark on a deck portion of the surgical staple cartridge that corresponds to a size of the clamped tissue. The method further comprises cutting the clamped tissue to form two severed end portions and stapling each severed end portion.

### BRIEF DESCRIPTION OF DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:
FIG. 1 is a perspective view of one form of a surgical cutting and stapling instrument with which various cartridges and end effector embodiments of one form of the present invention may be used;
FIG. 2 is an exploded view of an end effector embodiment of the present invention;
FIG. 3 is a perspective view of a staple cartridge embodiment of one form of the present invention;
FIG. 4 is a partial perspective view of the end effector portion of the instrument of FIG. 1 in an open position and prior to clamping a piece of tissue therein;
FIG. 5 is a plan view of a portion of an end effector embodiment clamping a vessel between the anvil and staple cartridge thereof;
FIG. 6 is a plan view of a portion of another end effector embodiment clamping a vessel between the anvil and staple cartridge thereof;
FIG. 7 is a perspective view of another surgical stapling cartridge embodiment of one form of the present invention;
FIG. 8 is a plan view of a portion of another end effector embodiment clamping a vessel therein; and
FIG. 9 is a partial perspective view of the end effector of FIG. 8 after the anvil has been moved to an open position and the tissue pieces removed therefrom.

### DETAILED DESCRIPTION

Certain exemplary embodiments will now be described to provide an overall understanding of the principles of the structure, function, manufacture, and use of the instruments and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the various embodiments of the present invention is defined solely by the claims. Reference throughout the specification to "various embodiments," "some embodiments," "one embodiment," or "an embodiment", or the like, means that a particular feature, structure, or characteristic described in connection with the embodiment is included in at least one embodiment. Thus, appearances of the phrases "in various embodiments," "in some embodiments," "in one embodiment", or "in an embodiment", or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation. Such modifications and variations are intended to be included within the scope of the various invention embodiments disclosed herein and their respective equivalents.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary instruments and methods are provided for performing laparoscopic and minimally invasive surgical procedures. However, the person of ordinary skill in the art will readily appreciate that the various methods and instruments disclosed herein can be used in numerous surgical procedures and applications including, for example, in connection with "open" surgical procedures. As the present Detailed Description proceeds, those of ordinary skill in the art will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device such as a trocar that has a working channel through which the end effector and elongated shaft of a surgical instrument can be advanced.

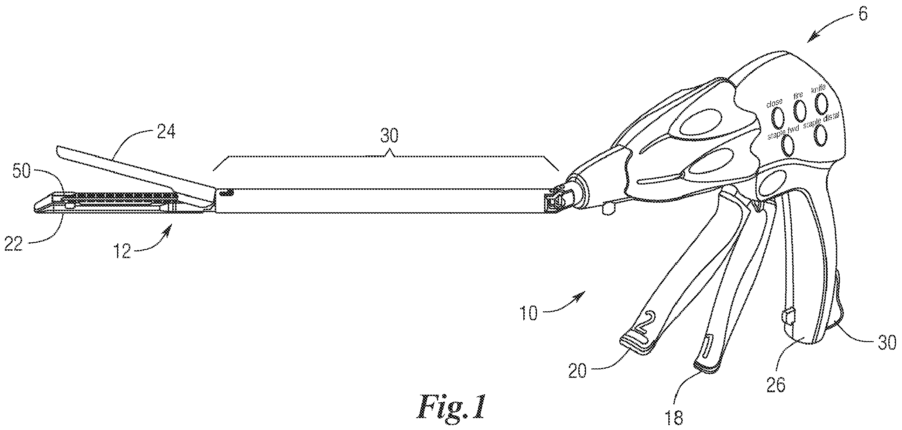

Turning to the Drawings wherein like numerals denote like components throughout the several views, FIG. 1 depicts one embodiment of a surgical stapling and severing instrument 10 that is capable of practicing various unique benefits of at least one form of the present invention. Various forms of the surgical instrument 10 are disclosed in U.S. Patent No. 7,753,904 entitled "Endoscopic Surgical Instrument With a Handle That Can Articulate With Respect to the Shaft", the entire disclosure of which is herein incorporated by reference. As such, the details concerning the construction and operation of that instrument not needed to understand the various embodiments and forms of the present invention will not be specifically repeated herein. The surgical instrument depicted in FIG. 1 is a motor driven or "powered instrument". As the present Detailed Description proceeds, the skilled artisan will appreciate that various unique and novel aspects of at least some of invention embodiments may also be effectively employed in connection with surgical stapling and severing instruments that employ mechanical (unpowered) systems for firing the staples and cutting tissue without departing from the spirit and scope of the present invention. Further, although various instrument embodiments have been depicted herein with a handheld "handle", the instruments disclosed herein may be adapted for receiving clamping and/or firing motions from a robot or similar device for applying actuation motions thereto. Thus, the scope of protection afforded to various end effectors and cartridge embodiments disclosed herein should not be limited in anyway to use solely with hand-held surgical instruments.

As can be seen in FIG. 1, one form of a surgical instrument 10 comprises a handle 6 that has an elongated tube assembly 30 that is operably attached thereto that is configured to transmit actuation motions to an end effector 12 that is attached to a distal end portion of the elongated tube assembly 30. The end effector 12 includes a channel 22 that is coupled to support various forms of staple cartridges of the present invention as will be discussed in greater detail below. An anvil 24 is movably supported relative to the channel 22 in response to opening and closing motions applied thereto by various portions of the elongated tube assembly 30.

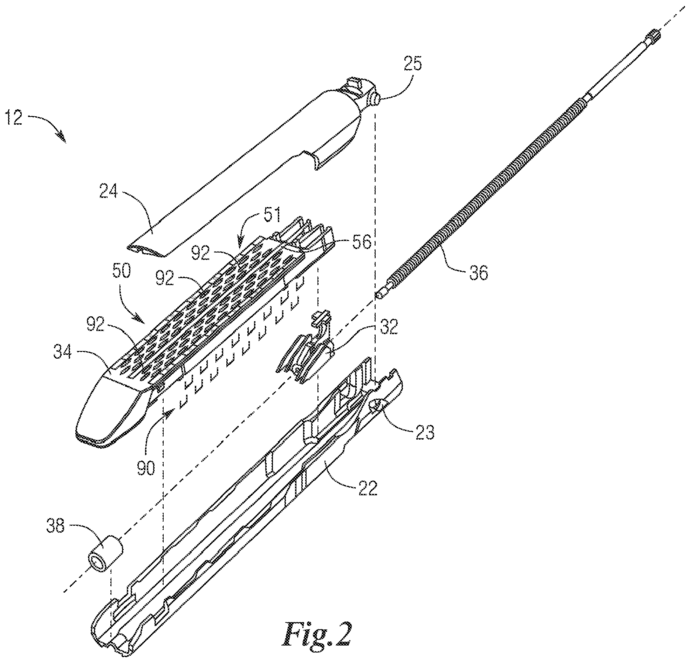

The handle 6 includes a pistol grip 26 toward which a closure trigger 18 may be pivotally drawn by the clinician to cause clamping or closing of the anvil 24 toward the staple channel 22 of the end effector 12. A firing trigger 20 is farther outboard of the closure trigger 18. As shown in FIG. 2, the end effector 12 may include, in addition to the previously mentioned channel 22 and anvil 24, a knife and sled driving member 32, a staple cartridge 50 that supports a plurality of unformed staples 90 therein, a helical screw shaft 36 and a bearing 38 that is attached to the channel structure 22. The anvil 24 may be pivotably connected to the channel 22 at a proximate pivot location. In one embodiment, for example, the anvil 24 includes laterally projecting pivot pins 25 at its proximal end that pivotally engage pivot apertures 23 formed near the proximal end of the channel 22. When the closure trigger 18 is actuated, that is, drawn in by a user of the instrument 10, the trunnions 25 of the anvil 24 may pivot within the pivot apertures 23 in the channel 22 about the pivot location into the clamped or closed position. If clamping of the end effector 12 is satisfactory, the operator may actuate the firing trigger 20, which activates a motor/transmission (not shown) in the handle 6 that applies rotary motion to the helical screw shaft 36 to cause the knife/sled driving member 32 to travel along the channel 22, thereby cutting tissue clamped within the end effector 12 and driving the unformed staples 90 into forming contact with the underside of the anvil 24. As used herein, the term "fire" with respect to the staples refers to the actions involved with driving the unformed staples 90 out of their respective staple pockets 92 within the staple cartridge and into forming contact with a corresponding portion of the anvil. As the present Detailed Description proceeds, the reader will appreciate, however, that at least some of the unique and novel aspects of the various invention embodiments may be advantageously employed in connection with a variety of other surgical staplers and surgical stapler instruments including those surgical stapling units configured for use with so-called disposable loading units such, for example, those devices disclosed in U.S. Patent Application Publication No. 2006/0011699 A1, entitled "Surgical Stapler With Universal Articulation and Tissue Pre-Clamp", the disclosure of which is herein incorporated by reference in its entirety. Accordingly, the scope of protection afforded to the various embodiments of the present invention should not be limited to use with one particular type of surgical instrument and/or end effector.

After the knife/sled driving member 32 has been driven to the distal end of the staple cartridge 50, the clinician releases the firing trigger 20 to enable the firing trigger 20 to return to an open position, which will result in the application of a rotary retraction motion to the knife/sled driving member 32 to cause it to move proximally to a starting position. Once the knife/sled driving member 32 has been moved to a starting position out of the staple cartridge 50, the clinician may unlock the closure trigger 18 by means of a release button 30 on the handle to permit the closure trigger 18 to move to the open position and thereby cause the anvil 24 to pivot open and release the divided and stapled tissue.

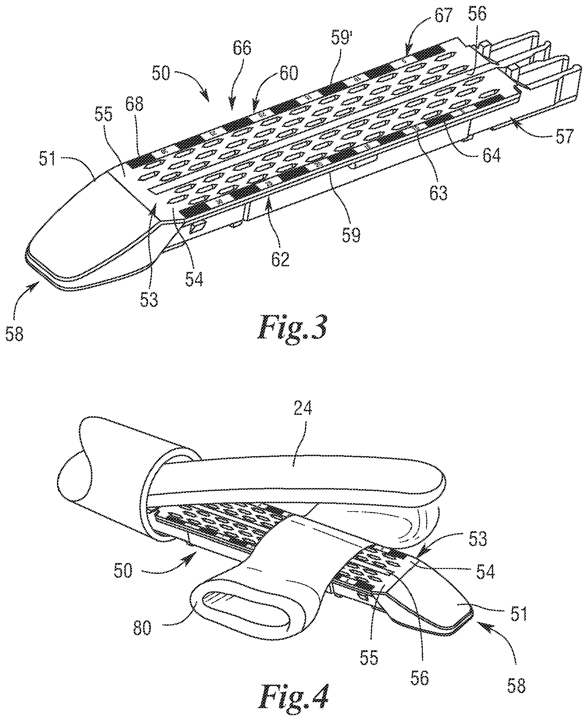

In the embodiments depicted in FIGS. 3 and 4, the staple cartridge 50 includes a cartridge body 51 that supports a plurality of unformed staples 90 therein. The cartridge body 51 has a deck 53 that has a centrally disposed slot 56 therein that divides the deck 53 into a first deck portion 54 and a second deck portion 55. The slot 56 accommodates the knife/sled driving member 32 (FIG. 2) as it is driven longitudinally within the cartridge body 51. In general, the slot 56 extends from a proximal end 57 to the distal end 58 of the cartridge body 51.

In various embodiments, measurement means generally represented as 60 is on at least one of the staple cartridge 50 and the anvil 24. In the embodiment depicted in FIGS. 3 and 4 for example, the measurement means 60 comprises a first series of measurement indicia 62 on the first deck portion 54 adjacent a first lateral edge 59 thereof. In various embodiments, the first series of measurement indicia 62 comprises a series of graduated measurement indicia 63 that are separated by a predetermined distance. The predetermined distance may correspond to a mark 64 that has a color that differs from the color of the deck 53. For example, for a staple cartridge 50 with a staple line length of 30mm, the first series of measurement indicia may extend from, for example, 0mm to 30mm or 32.5mm as shown. That is, each balck mark and white space therebetween may be approximately 2.5mm long. Other forms of graduated measurement indicia (e.g., a segment of a measurement scale, etc.) may be used.

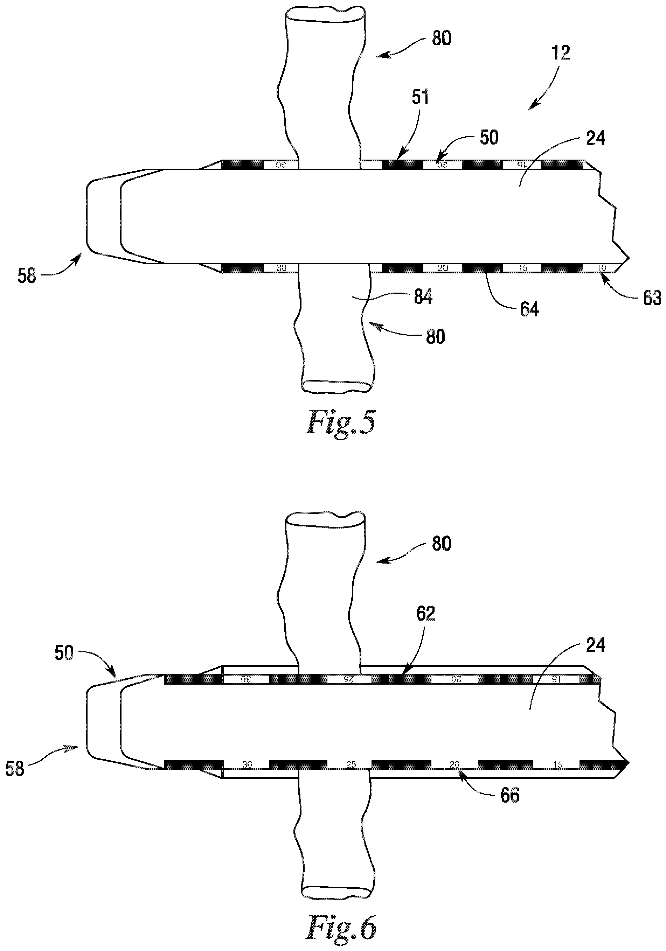

Also in the embodiment depicted in FIG. 3, the measurement means 60 further comprises a second series of measurement indicia 66 on the second deck portion 55 adjacent a second lateral edge 59' thereof. In various embodiments, the second series of measurement indicia 66 comprises a series of graduated measurement indicia 67 that are separated by a predetermined distance. The predetermined distance may correspond to a mark 68 that has a color that differs from the color of the deck 53. The color of mark 68 may correspond to the color of mark 64 or it may not. The length of each mark 68 may correspond to the length of each mark 64. Other forms of graduated measurement indicia (e.g., a segment of a measurement scale in millimeters and/or inches, etc.) may be used. In other embodiments, only one series of measurement indicia is provided. In at least one embodiment, the series of measurement indicia only performs a measurement function. That is, the measurement indicia in at least one embodiment comprises indicia that does not perform any other function on the staple cartridge and/or anvil other than to provide the user with a means to assess the size of a vessel 80 clamped between the deck 53 and the underside of the anvil 24. See, e.g., FIGS. 4 and 5. Those of ordinary skill in the art will appreciate that such arrangements of the measurement indicia 62, 66 enable the clinician to assess the diameter or other size of the tissue that is clamped in the end effector 12 when viewing the end effector 12 from above (as shown, for example, in FIGS. 5 and 6). In the embodiment depicted in FIG. 6, the measurement indicia series 62, 66 are provided on the anvil 24. As with the above-described embodiment, only one of the measurement indicia series may be employed on the anvil 24. The measurement indicia series may substantially encompass the entire upper surface of the anvil 24 or just portions thereof.

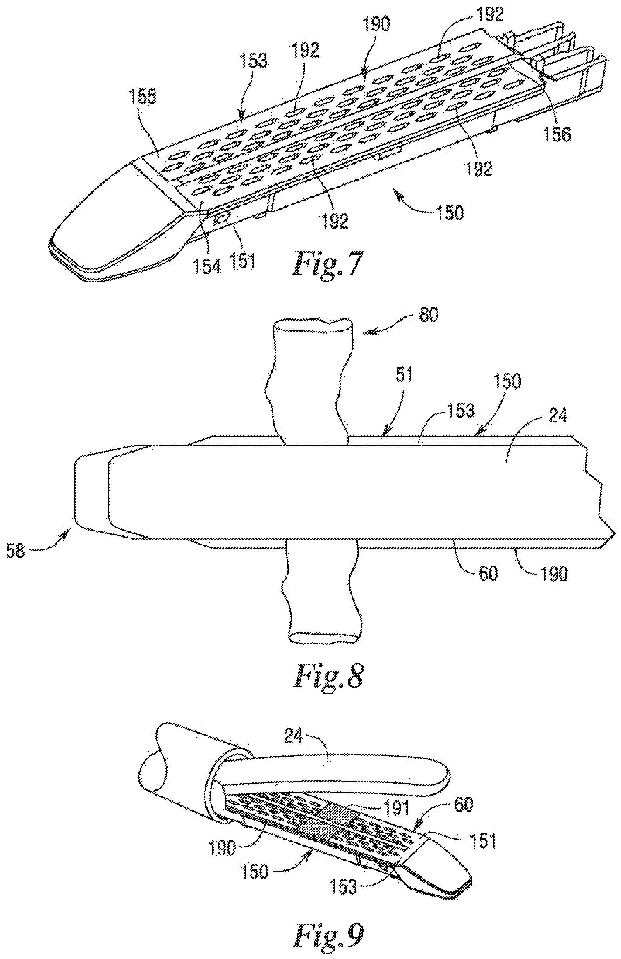

FIG. 7 depicts another staple cartridge embodiment 150 that is substantially identical to the staple cartridge 50 described above, except for the differences discussed below. The staple cartridge 150 includes a cartridge body 151 that supports a plurality of unformed staples in the staple pockets 192 therein. The cartridge body 151 has a deck 153 that has a centrally disposed slot 156 therein that divides the deck 153 into a first deck portion 154 and a second deck portion 155. In this embodiment, the measurement means 60 comprises a mark-forming material 190 on the deck 153. In use, after the vessel 80 has been clamped between the anvil 24 and cartridge 150 as shown in FIG. 9, the portion 191 of the mark-forming material 190 that has been compressed by the tissue changes color or changes in shade or tint (e.g., darkness of color) such that the portion of the mark-forming material 190 that has been compressed or merely contacted by the tissue may be distinguished from the portions of the mark-forming material 190 that have not been compressed or otherwise contacted by the clamped tissue. Such arrangement thereby provides the clinician with feedback that could be used to assess the transected vessel for hemostatic purposes. Also, such arrangement provides an indication of where the tissue was on the cartridge deck for production inquiry investigations. In addition, such arrangement also provides the user with an indication of where the staples were fired directly into the anvil without penetrating through tissue.

In various embodiments, the mark-forming material 190 may be applied as a coating on the cartridge deck. In other embodiments, the mark-forming material 190 may comprise a substrate that is attached to the cartridge deck or jaw portion of the non-stapler form of end effector. The mark forming material may comprise, for example, a material that contains an indicator that identifies the tissue based on its PH or chemical composition. For example, the mark-forming material 190 may comprise material that changes color or tint or shade of the same color when merely contacted by tissue without requiring the tissue to be compressed thereon.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

While this invention has been described as having exemplary designs, the present invention may be further modified within the spirit and scope of the disclosure. This application is therefore intended to cover any variations, uses, or adaptations of the invention using its general principles. Further, this application is intended to cover such departures from the present disclosure as come within known or customary practice in the art to which this invention pertains.

## Claims

1. A surgical staple cartridge comprising:
a cartridge body operably supporting a plurality of surgical staples therein, said cartridge body having a proximal end and a distal end and a deck extending therebetween; and
measurement means on said deck for determining a size of tissue placed thereon.

2. The surgical staple cartridge of claim 1 wherein said measurement means comprises at least one series of measurement indicia on said deck.

3. The surgical staple cartridge of claim 2 wherein said deck has first and second lateral edges and wherein said at least one series of measurement indicia comprises:
a first series of graduated measurement indicia on said deck adjacent said first lateral edge thereof; and
a second series of graduated measurement indicia on said deck adjacent said second lateral edge thereof.

4. The surgical staple cartridge of claim 2 wherein at least one said series of measurement indicia comprises a series of marks that are separated from each other by a predetermined distance.

5. The surgical staple cartridge of claim 4 wherein said deck has a first color and wherein each said mark has a color that differs from said first color.

6. The surgical staple cartridge of claim 3 wherein said first series of measurement indicia comprises a first series of first marks that are separated from each other by a first predetermined distance and wherein said second series of measurement indicia comprises a second series of second marks that are separated from each other by a predetermined distance.

7. The surgical staple cartridge of claim 6 wherein said deck has a deck color and wherein each said first and second mark has a first color that differs from said deck color.

8. The surgical staple cartridge of claim 1 wherein said means comprises a material on said deck that has a first color that changes to a different color where contacted by tissue.

9. The surgical staple cartridge of claim 1 wherein said means comprises means for forming a mark on said deck that substantially corresponds to the size of the tissue placed thereon when contacted by the tissue.

10. The surgical staple cartridge of claim 9 wherein means for forming a mark comprises pressure sensitive material on said deck.

11. The surgical staple cartridge of claim 10 wherein said material comprises a pressure sensitive substrate attached to said deck.

12. A surgical end effector for use with a surgical instrument, said surgical end effector comprising:
an elongated channel operably couplable to the surgical instrument;
a staple cartridge having a cartridge body operably supported in said elongated channel, said cartridge body having a deck surface substantially split into a first deck portion and a second deck portion by a longitudinal slot extending therebetween, said cartridge body operably supporting a first plurality of unformed staples therein corresponding to said first deck portion and a second plurality of unformed staples corresponding to said second deck portion;
a tissue cutting member operably supported in said cartridge body for axial advancement in said longitudinal slot upon application of a cutting actuation motion thereto by the surgical instrument;
an anvil supported for movable travel toward and away from said deck surface in response to opening and closing motions applied thereto by the surgical instrument; and
measurement means on at least one of said deck surface and said anvil for determining a size of tissue placed thereon.

13. The surgical end effector of claim 12 wherein said means comprises:
a first measurement means on said first deck portion for assessing a size of tissue clamped between said anvil and said deck surface when viewed from above the deck surface; and
a second measurement means on said second deck portion for assessing the size of the tissue clamped between said anvil and said deck surface when viewed from above the deck surface.

14. The surgical end effector of claim 13 wherein said deck has first and second lateral edges and wherein said first measurement means comprises a first series of graduated measurement indicia on said deck adjacent said first lateral edge thereof and wherein said second measurement means comprises a second series of graduated measurement indicia on said deck adjacent said second lateral edge thereof.

15. The surgical end effector of claim 14 wherein said first series of measurement indicia comprises a first series of first marks that are separated from each other by a first predetermined distance and wherein said second series of measurement indicia comprises a second series of second marks that are separated from each other by a predetermined distance.

16. The surgical end effector of claim 15 wherein said deck has a first color and wherein each said first and second mark has a color that differs from said first color.

17. A method for severing and stapling tissue comprising:
clamping a piece of the tissue between a surgical staple cartridge and an anvil;
forming a mark on a deck portion of the surgical staple cartridge that corresponds to a size of the clamped tissue;
cutting the clamped tissue to form two severed end portions; and
stapling each severed end portion.

18. The method of claim 17 further comprising assessing the size of the clamped tissue.

19. The method of claim 18 wherein said assessing comprises viewing the mark on the deck portion.

20. The method of claim 19 wherein said viewing comprises viewing the mark on the deck portion from above the deck portion.
